# EUROPEAN PATENT APPLICATION

(11) **EP 2 784 511 A1**
(43) Date of publication of application: **01.10.2014**
(21) Application number: 13161489.3
(22) Date of filing: 27.03.2013
(51) Int. Cl.: G01N 33/574

(54) **Integrin alpha-v-beta6 for diagnosis/prognosis of colorectal carcinoma**

(71) Applicant: Universität Zürich, 8006 Zürich (CH)
(72) Inventor: The designation of the inventor has not yet been filed
(74) Representative: Schulz Junghans Patentanwälte PartGmbB

(57) **Abstract**

The invention relates to a method for diagnosis of colorectal carcinoma in a patient, comprising detecting, in a sample obtained from said patient, an indicator protein selected from the group comprised of integrin subunit alpha(v) and integrin subunit beta(6) or their heterodimer.

## Description

Colorectal carcinoma is the third most frequent tumour worldwide. Though colorectal carcinoma can be sufficiently treated and often even be cured as long as the tumour is small and detected at an early stage, the prognosis of patients suffering from metastatic colorectal carcinoma is unsatisfactory, as many do not survive beyond 5 years.

To date, however, there are no reliable markers available, particularly serum markers. The common tumour markers for colorectal carcinoma include carcinoembryonic antigen (CEA) and CA19-9. However, these tumour markers are not specific for colorectal carcinoma. Furthermore, they are also not sufficiently sensitive for the definitive diagnosis of the disease and can serve only as progress markers during the course of disease. They can be highly elevated in patients without colorectal carcinoma and can even be at normal levels in patients with highly advanced colorectal carcinoma.

Considering the crucial importance of an early diagnosis for the outcome and prognosis of colorectal carcinoma patients, there is a considerable medical need of soluble biomarkers for non-invasive, economical and high-throughput diagnostics of colorectal carcinoma. Such biomarkers may be helpful in i) improving risk prediction and prognostics of colorectal carcinoma, ii) providing earlier and more reliable diagnosis of colorectal carcinoma, and iii) enabling to monitor progression and regression of colorectal carcinoma under treatment.

Hence, the objective of the present invention is to provide biomarkers for diagnosis of colorectal carcinoma that improve on the state of the art. This objective is attained by the subject matter of the independent claims.

By analyzing serum of patients, it was surprisingly found that integrin alpha(v)beta(6) (integrin αvβ6) can serve as a biomarker of colorectal carcinoma. Serum samples were obtained from healthy control patients and patients with colorectal carcinoma. Levels of integrin αvβ6 were analysed by enzyme-linked immunosorbent assay (ELISA). Increased levels of integrin αvβ6 were found in the colorectal carcinoma patients group, demonstrating the diagnostic relevance of integrin αvβ6.

The sequences of the integrin subunits alpha(v) and beta(6) are defined by UniProt No. P06756 and UniProt No. P18564.

The integrin subunits alpha(v) and beta(6) form the heterodimer integrin alpha(v)beta(6) (integrin αvβ6).

According to a first aspect of the invention, a method is provided for diagnosis or prognosis of colorectal carcinoma in a patient, or for assessing a patient's risk of developing or having developed colorectal carcinoma, said method comprising detection of an indicator protein selected from the group comprised of integrin subunit alpha(v), integrin subunit beta(6) and the heterodimer of integrin alpha(v) beta (6). In some embodiments, the method is performed ex *vivo* on a sample obtained from the patient, particularly a blood sample.

Any known method may be used for the detection of the indicator protein (integrin subunit alpha(v), integrin subunit beta(6) or the heterodimer of integrin alpha(v) beta (6)) in a sample such as body fluids. Methods considered are, by way of non-limiting example, chromatography, mass spectrometry (and combinations thereof), enzymatic assays, electrophoresis and antibody based assays (like ELISA, RIA, EIA, CLIA (see Zhou et al, J Zhejiang Univ Sci B. 2005 Dec;6(12):1148-52 and references cited therein), CEDIA (see Hamwi et al., Am J Clin Pathol. 2000 Oct;114(4):536-43 and references cited therein), CMIA(see Berry et al., Clin Chem. 1988 Oct;34(10):2087-90 and references cited therein), MEIA (see Rodrigues et al., Mem Inst Oswaldo Cruz. 2009 May;104(3):434-40 and references cited therein), FPIA (see Stricher et al., Biochem J. 2005 Aug 15;390(Pt 1):29-39 and references cited therein), GLORIA (see Armenta et al., Trends in Analytical Chemistry, Vol. 27, No. 4, 2008 and references cited therein), microarray analysis, fully-automated or robotic immunoassays and latex agglutination assays).

Modern bio-analytical methods facilitate the direct isolation and detection of proteins, i.e. without prior binding of the protein to an antibody or other selective ligand. The sample may be submitted, for example, to one or more runs of chromatography, followed by mass spectroscopy or multi-dimensional mass spectroscopy analysis (HPLC-MS; LC-MSMS) to identify the relevant protein component of a sample quantitatively. Sample preparation steps to remove, for example, cells or non-relevant blood components may precede the analysis.

In the context of the present specification, the term "amount" also refers to concentration. It is evident that from the total amount of a compound of interest in a sample of known size, the concentration of the compound can be calculated, and vice versa.

In the context of the present invention the term "sample" refers to any component of the sample independently from separation of the components, and encompasses the protein contents of a blood sample processed by several separation or washing steps.

A variety of sampling methods offer themselves to practice the invention. In some embodiments, the sample is a blood sample, particularly a peripheral blood sample.

Some embodiments include the use of one or more ligand(s) specifically reactive to an indicator protein selected from the group comprised of integrin subunit alpha(v) and integrin subunit beta(6).

A ligand according to any aspect or embodiment of the invention may be any molecule that binds to the indicator protein selected from the group comprised of integrin subunit alpha(v) and integrin subunit beta(6) with high affinity and specificity.

High affinity in the context of the present specification refers to the dissociation constant of the binding of the ligand to the indicator protein, wherein the dissociation constant is 10⁻⁷, 10⁻⁸ or 10⁻⁹ mol/l or less and wherein the ligand does not bind to control proteins with unrelated structural features. Control proteins are, by the way of non-limiting example, plasma proteins such as albumins, globulins, lipoproteins, fibrinogens, prothrombin, acute phase proteins, tumour markers such as CEA, CA19-9 or AFP and transferrin.

High specificity in the context of the present specification refers to the ratio of properly detected indicator protein and the sum of all detected polypeptides, wherein the ratio is 80 %, 85 %, 90 %, 95 %, 99 % or 99.9 %.

In one embodiment, the ligand is an antibody.

In one embodiment, the ligand is a gamma immunoglobulin.

In one embodiment, the ligand is a monoclonal antibody binding to or raised against integrin subunit alpha(v) or integrin subunit beta(6).

One non-limiting example of such monoclonal antibody is the antibody against integrin subunit beta(6) from USCNK Life Sciences Inc., Wuhan, China (Enzyme-linked Immunosorbent Assay Kit for Integrin β6, E92099Hu).

In one embodiment, the monoclonal antibody produced by the hybridoma ATCC HB12382 (US 7,150,871) is employed as a ligand.

In one embodiment the ligand is a Fab fragment of an antibody. A Fab fragment is the antigen-binding fragment of an antibody, specifically reactive to the indicator protein.

In another embodiment the ligand is a single-domain antibody (sdAb or Nanobody), for instance a single-chain variable fragment (scFv) antibody selected by phage display methods, or a camelide antibody, specifically reactive to the indicator protein.

Alternatively, a ligand employed in practising the invention may be a molecule engineered or selected to demonstrate a high specificity and affinity for the indicator protein. Such engineered or selected molecule may be an antibody-like polypeptide or synthetic antibody selected by phage-display (see Pini et al., J.Biol. Chem. (1998) 273, 21769-21776) and references cited therein, or by other evolutionary selection methods). Alternatively, the ligand may be a polynucleotide or spiegelmer with high selectivity for the indicator protein (e.g. SELEX, see Ellington & Szostak, Nature 346, 818-822 and references cited therein).

An antibody like molecule may be, but is not limited to, a repeat protein, such as a "designed ankyrin repeat protein" (Molecular Partners, Zürich).

In another embodiment of the invention the ligand is a ligand of integrin avb6, like LAP-TGF-β, osteopontin, fibronectin or ADAM family members.

In another embodiment of the invention the ligand is an antagonist of the indicator protein, such as a small molecule or a cyclic peptide (see EP 1754714 A1).

According to some embodiments, the method of the invention further comprises the steps of contacting, in a first contacting step, the sample with a first ligand specifically reactive to the indicator protein, and determining, in a quantification step, the amount of the indicator protein bound to the ligand.

In some embodiments, the method thus comprises the following steps:
- blood or other fluid sample obtained from a patient is contacted, optionally after a work-up for removal of irrelevant components, with a surface, as a consequence of which the components of the sample adhere to the surface (e.g. through charge interactions or hydrophobic interactions);
- optionally, a blocking reagent is added to block any surface that remains uncoated by the sample and thus may lead to binding of later antibody or other ligands. Examples for such blocking reagents are detergents (e.g. Triton X-100 or Tween) or proteins (e.g. bovine serum albumin, non-fat dry milk, casein or fish gelatine).
- a first ligand specifically reactive to the indicator protein (integrin subunit alpha(v), integrin subunit beta(6) or their heterodimer) binds to the indicator protein bound to the surface;
- a ligand specifically reactive to the first ligand is contacted with the first ligand, and the amount of the indicator protein bound to the first ligand is determined;
- optionally, a washing step is inserted between the first and the second step to remove any sample components that did not interact with the surface. Another washing step may follow the second contacting step and precede the determination of indicator protein.

In one embodiment, the ligand specifically reactive to the first ligand is an antibody. If the first ligand is an antibody, the ligand specifically reactive to the first ligand may be a polyclonal or monoclonal antibody specific for the first antibody or fragments thereof, such as the Fc or Fab region. In one embodiment, the antibody specifically reactive to the first antibody is attached or specifically attachable to an enzyme activity or a detectable label.

In a classical ELISA the ligand specifically reactive to the first ligand is an antibody specifically reactive to the Fc part of human IgG or IgM and carries an enzymatic activity or label. Typically the antibody specifically reactive to the first antibody is attached to horse radish peroxidase or a fluorescent label.

In embodiments where the first ligand is an antibody that is labelled with biotin, the ligand specifically reactive to the first ligand is streptavidin or avidin that tightly binds to the biotin attached to the first ligand. In such embodiments, avidin or streptavidin is then attached to an enzyme activity or a detectable label.

In some embodiments, the detection of the indicator protein is performed in a homogenous immunoassay, such as EMIT (Beresini et al., Clin Chem. 1993 Nov;39(11 Pt 1):2235-41 and references cited therein) or a FRET-based homogenous immunoassay (Kreisig et al., Anal Chem. 2011 Jun 1;83(11):4281-7 and references cited therein).

In the FRET-based homogenous immunoassay, the sample is spiked with a fixed amount of peptide derivatized with a fluorophore outside the antibody recognition site (donor peptide). This peptide has a lower affinity to the antibody recognition site than the indicator protein. If no indicator protein is present, the donor peptide binds to the acceptor-labelled antibody, quenching the donor emission intensity due to FRET. If the indicator protein is present, the donor peptide competes with the analyte, resulting in a higher signal due to the partially released donor peptide.

In some embodiments, the ligand has a fluorescent or luminescent quality and determining the amount of indicator protein is performed by fluorescence-activated cytometry. A fluorescent or luminescent quality in the sense of the invention refers to the ability of the first ligand to emit light after excitation by light (of a shorter wavelength than the excitation wavelength).

In some embodiments, the method according to the invention will involve the comparison of the determined amount of protein with a standard. An absolute measurement may achieve the desired accuracy and validity, for example if a reproducible determination of binding can be attained, as is the case for surface plasmon resonance (SPR) assays with a properly calibrated SPR device, or spectroscopic read-outs in HPLC, where the integral of the chromatogram is proportional to the amount of analyte.

In some embodiments, the ligand is attached to a surface. A surface is the surface of a microtiter well, a lab-on-a-chip device (LOC, microfluidics analysis), a microarray or the matrix material of a lateral flow immunoassay.

A ligand is attached, in the context of the present invention, if the ligand is bound to the surface by covalent or non-covalent bonds so that the attachment withstands repeated washing steps with aqueous buffer of pH 5 - 8. One example of attachment is a covalent bond, for example by ester or amide linkage or disulfide bridge; another is the streptavidin-biotin bond or simple adsorption of proteins to polymer surfaces. Ligands may be attached to gold surfaces, for example by sulfide groups.

In some embodiments the surface is the surface of a surface plasmon resonance or quartz microbalance chip, which will allow direct measurement of the amount of indicator protein attached to the ligand by means of the changes effected in the physical properties of the chip. The amount of indicator protein is thus determined directly.

According to some embodiments, the quantification step further comprises the use of a second ligand with specificity for the targeted indicator protein. The second ligand binds to a different surface section of the target in order to allow for the first and second ligand to be able to bind concomitantly.

The second ligand may carry an enzymatic activity or label, such as a peroxidase, to allow for fitting the method into the well-established ELISA formats. Other labels may be, but are not limited to, a phosphatase or luciferase activity, or the detectable label is a radioactive label or a gold particle.

Likewise, the second ligand may have an optically detectable quality such as a colour label, a fluorescent, a phosphorescent or a luminescent quality.

In general, suitable labels are chromogenic labels, i.e. enzymes which can be used to convert a substrate to a detectable colored or fluorescent compound, spectroscopic labels, e.g. fluorescent labels or labels presenting a visible color, affinity labels which may be developed by a further compound specific for the label and allowing easy detection and quantification, or any other label suitable to standard ELISA formats. Examples for fluorescent labels or labels presenting a visible color include, without being restricted to, Fluorescein isothiocyanate (FITC), rhodamine, Allophycocyanine (APC), Peridinin chlorophyll (PerCP), Phycoerithrin (PE), Alexa Fluors (Life Technologies, Carlsbad, CA, USA), Dylight fluors (Thermo Fisher Scientific, Waltham, MA, USA) ATTO Dyes (ATTO-TEC GmbH, Siegen, Germany), BODIPY Dyes (4,4-difluoro-4-bora-3a,4a-diaza-s-indacene based dyes) and the like.

An optically detectable label or enzyme activity may be detected on a surface.

In some embodiments, the method according to the invention thus comprises the following steps:
- in a first contacting step, a blood or other fluid sample obtained from a patient is contacted, optionally after a work-up for removal of irrelevant components, with a surface onto which a first ligand specifically reactive to the indicator protein (integrin subunit alpha(v), integrin subunit beta(6) or their heterodimer) is attached;
- in a second contacting step, a second ligand is contacted with the surface, and the amount of the second ligand is determined.
- Optionally, a washing step is inserted between the first and the second contacting step to remove any sample components other than the indicator protein. Thereby, the sample is removed along with any component previously contained therein that had not been bound by the first ligand. Another washing step may follow the second contacting step and precede the determination of the second ligand.

According to one embodiment, the first and the second ligand are attached to the respective components of a system that allows to determine a quantitative measure of proximity-paired partners, such as the "Amplified Luminescent Proximity Homogeneous Assay (ALPHA)" commercialized by Perkin Elmer, whereby singlet oxygen is transferred from a donor partner to an acceptor, resulting in emission of light, if the two partners are within 200 nm distance. Such arrangement allows for the practice of the invention in solution.

According to another embodiment, a liquid volume of sample is placed onto a sample area composed in such way as to provide capillary forces that act to transport at least a part of the sample towards an analysis area. Between the sample area and the analysis area, or as part of the sample area, a conjugate area is provided. First ligands specific for the indicator protein are provided, without attachment to the matrix, in the conjugate area, and upon binding to indicator protein in the sample, these first ligands are drawn with the capillary flow into the analysis area. The first ligands according to this embodiment of the invention are conjugated to latex or gold microparticles or enzymatically active proteins. Second ligands are attached to the analysis area, forming a line. Upon binding of indicator proteins (with first ligands attached) to the second ligands, the resulting "sandwiches" of second ligand, indicator protein and first ligand form a line, which may be visible as such (if particles are attached to the first ligands), or may be rendered visible by providing a substrate for an enzymatic activity attached to the first ligand. This embodiment is referred to as a lateral flow assay.

In some embodiments, the level of indicator protein(s) in the sample is compared to the corresponding levels in (a) sample(s) of (a) healthy subject(s). An altered level compared to the level in the sample of a healthy subject is indicative of a disease or of the risk to develop the disease.

According to one embodiment, the measured levels of indicator protein (integrin subunit alpha(v), integrin subunit beta(6) or their heterodimer) indicates whether an individual is suffering from colorectal carcinoma or has an increased risk of developing such disease. The terms used in this context, i.e. "non-increased level", "not elevated level", "increased levels" and "decreased levels" are known to, or can be determined by the person skilled in the art according to, for example, the procedure outlined in the following paragraphs.

In some embodiments, the level of the indicator protein is compared to a defined threshold. In one embodiment, the threshold of the indicator protein is defined as between 2 and 10 ng avb6/ml. In one embodiment, the threshold of the indicator protein is defined as between 3 and 8 ng avb6/ml. In one embodiment, the threshold of the indicator protein is defined as 4 ng avb6/ml. Patients having a serum or plasma level of integrin avb6 > 2 ng/ml, > 3 ng/ml or > 4 ng/ml show an increased likelihood of suffering from metastatic disease, an undifferentiated tumor, a further advanced tumor and lower survival expectancy beyond 5 years. A serum level of integrin avb6 > 2 ng/ml, 3 ng/ml, or > 4 ng/ml can serve as an indicator for advanced disease and a prognostic marker for shorter survival of the respective patients.

The person skilled in the art is able to determine actual values corresponding to "non-increased level", "not elevated level", "increased level" and "decreased level" for the relevant biochemical markers. For example, the levels may be assigned according to percentiles of the levels observed in a representative sample of apparently healthy individuals, typically below an age of 50 years. The sample should be of sufficient size to yield statistically significant outcomes (for example, at least 100, more preferably at least 500, most preferably at least 1000 individuals). A non-increased level may correspond, by way of example, to the maximum level observed in the 97.5 % percentile of healthy individuals. Alternatively, the levels may be determined as "normal ranges" as known in the state of the art.

The levels may also be determined or further refined by studies performed on individuals by comparing indicator protein levels of apparently healthy individuals with individuals suffering from colorectal carcinoma. Such studies may also allow to tailor the levels according to the type of disease or/and certain patient sub-groups, e.g. elderly patients, patients undergoing medication or patients with a certain lifestyle.

The value of the levels considered as "increased" or "decreased" may also be chosen according to the desired sensitivity or specificity (stringency) of exclusion. The higher the desired sensitivity, the lower is the specificity of exclusion and vice versa. In the above example, the higher the percentile chosen to determine each level, the more stringent is the exclusion criterion, i.e. less individuals would be considered "individuals at risk".

The method of the invention also allows for the determination of the risk or the likelihood of an individual of suffering from colorectal carcinoma. In the context of the present invention, the terms "risk" or "likelihood" relate to the probability of a particular patient to develop colorectal carcinoma. The grade of risk can be decreased, non-increased, increased, or highly increased. "Non-increased risk" or "small likelihood" means that there is apparently no or very little increment in risk of suffering from or of developing colorectal carcinoma compared to the average risk of a healthy individual.

The degree of risk is associated with the level of indicator proteins. A non-altered level of indicator proteins indicates non-increased risk, an altered level of indicator proteins indicates an increased risk, and a highly altered level of indicator proteins indicates a highly increased risk.

If the level of indicator protein (integrin subunit alpha(v), integrin subunit beta(6) or their heterodimer) is altered, the patient might be recommended to undergo further diagnostic procedures such as colonoscopy, or a primary treatment or secondary interventions like the initiation of therapeutic life-style changes, drug therapy depending on risk factor constellation and reviews at regular intervals. If the diagnostic method of the present invention indicates an increased or highly increased risk, that information can be used to recommend further treatment of the individual.

According to a second aspect of the invention, the use of a device for diagnosis of colorectal carcinoma is provided, wherein the device comprises a surface, and said surface comprises a ligand specifically reactive to the indicator protein (integrin subunit alpha(v), integrin subunit beta(6) or their heterodimer). Such surface may be an ELISA plate, a LOC device, a surface plasmon resonance chip, a surface of a quartz microbalance sensor or the matrix material of a lateral flow immunoassay.

According to yet another aspect of the invention, the use of a kit of parts is provided for diagnosis of colorectal carcinoma in a patient, or for assessing a patient's risk of developing or having developed colorectal carcinoma, said kit comprising a first ligand and a second ligand, both ligands being specifically reactive to an indicator protein selected from integrin subunit alpha(v), integrin subunit beta(6) and their heterodimer.

According to one embodiment of this aspect of the invention, the first ligand is attached to the surface of a microtiter plate, and the second ligand comprises an enzymatic activity or a luminescent activity.

In some embodiments, the ligand is a monoclonal antibody, a single chain antibody or a single domain antibody.

The finding on which the present invention is based provides evidence that integrin αvβ6 may serve as a biomarker of colorectal carcinoma. Serum levels of integrin αvβ6 are significantly elevated in patients suffering from colorectal carcinoma. Serum levels of integrin αvβ6 in healthy control patients are at or below the level of detectability.

In principle, biomarkers indicating the onset of colorectal carcinoma would be good indicators of disease. Compared to conventional risk predictors such a marker of existing disease should also allow more reproducible absolute risk prediction in different populations than risk factors of which the penetrance is strongly influenced by environmental factors.

In one embodiment, the method of the invention is used for monitoring the progression and regression of colorectal carcinoma upon treatment. As yet progression and regression of colorectal carcinoma can only be assessed by imaging techniques. In one embodiment, the method of the invention is used to monitor treatment success by simple blood testing. The availability of such monitoring blood is of advantage not only for clinical management of patients but also for novel drug development, because such biomarker can be used as surrogate marker in phase 3 trials without clinical endpoints.

In one embodiment, the method of the invention is used in the acute diagnosis and treatment strategy of colorectal carcinoma. As yet this diagnosis is possible either by radiologic imaging, such as CT-scan or MRI-scan or by endoscopy. However, these procedures can have severe side-effects, in example due to radiation or the risk of gut perforation or wounding.

In one embodiment, the method of the invention is used to predict the tumour stage according to the TNM classification as well as the prognosis of patients suffering from colorectal carcinoma. So far, there is no marker available that is able to predict the tumour stage as well as prognosis and survival of colorectal carcinoma patients appropriately. Nevertheless, such information would be extremely helpful in the management of colorectal carcinoma patients.

In one embodiment, the method of the invention is used to predict whether certain colorectal carcinoma associated mutations are present within the tumour tissue of the patients, namely mutations within the gene encoding the epidermal growth factor receptor, the K-Ras gene and the B-Raf gene. As yet these diagnoses are possible only by histopathologic and/or molecular genetic assessment of tumour tissue specimen taken by endoscopy or surgery. However, these procedures can have severe side-effects, in example due to the risk of gut perforation or wounding, and are also highly expensive.

Measuring the presence of integrin αvβ6 in the blood is a cheap, fast and non-invasive alternative. The biomarkers that are currently available and are associated with the onset of colorectal carcinoma, such as carcinoembryonic antigen (CEA) or CA19-9, are not sufficiently specific and sensitive for the above described purposes. Hence, the present invention is not only useful to identify patients at risk but also to monitor interventions and to obtain information that plays a critical role for the further treatment of the patients.

The invention is further illustrated by the following examples and the figures, from which more advantages and embodiments of the inventions may be derived:

### Brief description of the figures

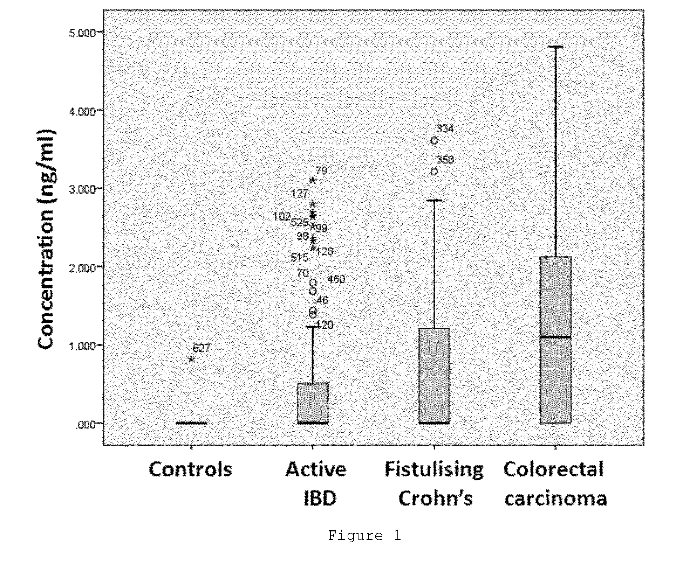

- Fig. 1: Serum levels of integrin αvβ6 are increased in patients with colorectal carcinoma. Serum levels of integrin αvβ6 are higher in patients with colorectal carcinoma than in patients with active inflammatory disease, patients with fistulising Crohn's disease or in healthy control patients. ELISA analysis using serum samples from 10 healthy control patients, 20 patients with Crohn's disease featuring an inflammatory disease type (B1) and 20 CD patients featuring a fistulising disease course (B3). Additionally, avb6-integrin serum levels were detected in an unselected cohort of 80 patients with colorectal cancer (CoCa) (any T, any N, any M status).

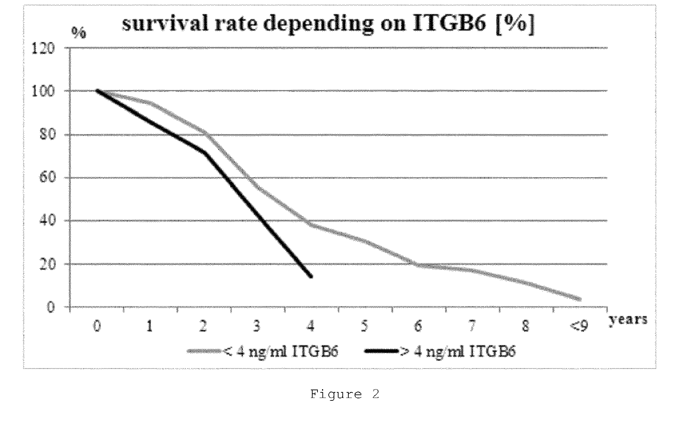

- Fig. 2: Serum levels of integrin avb6 > 4 ng/ml are a prognostic factor for survival. Patients featuring a serum level of integrin avb6 > 4 ng/ml show a shorter survival rate than patients with a serum level of integrin avb6 < 4 ng/ml.

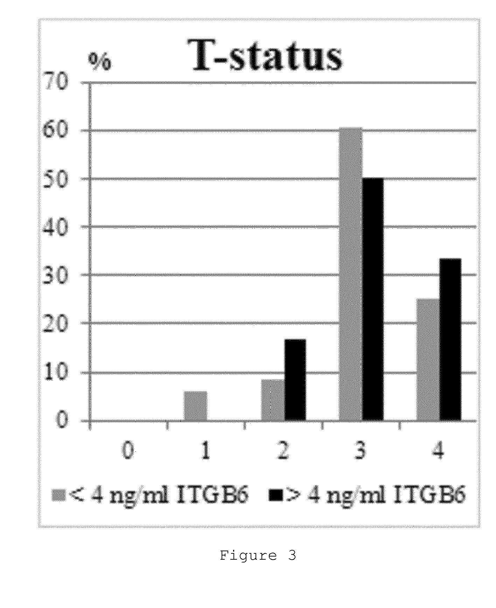

- Fig. 3: Serum levels of integrin avb6 > 4 ng/ml are a prognostic factor for tumour stage. Patients featuring a serum level of integrin avb6 > 4 ng/ml show a higher T-status than patients with a serum level of integrin avb6 < 4 ng/ml.

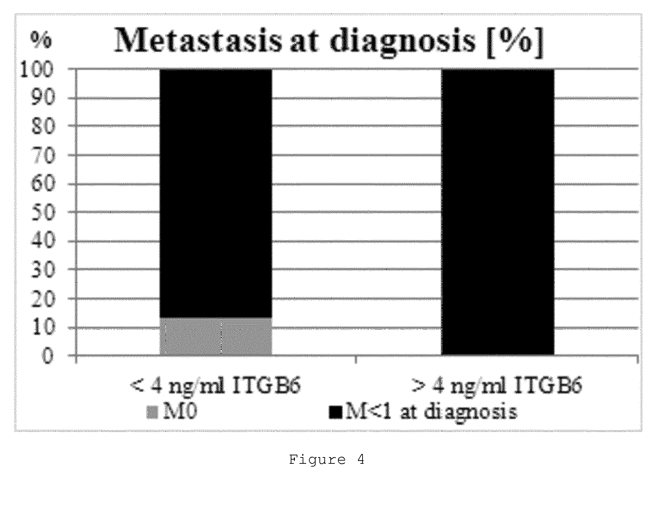

- Fig. 4: Serum levels of integrin avb6 > 4 ng/ml are a prognostic factor for tumour metastasis. Patients featuring a serum level of integrin avb6 > 4 ng/ml show always metastasis in contrast to patients with a serum level of integrin avb6 < 4 ng/ml.

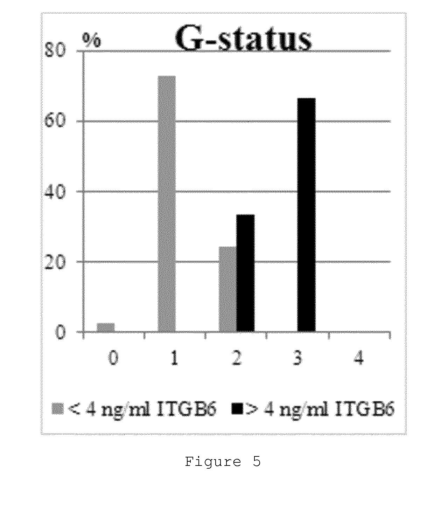

- Fig. 5: Serum levels of integrin avb6 > 4 ng/ml are a prognostic factor for differentiation of the tumour tissue. Patients featuring a serum level of integrin avb6 > 4 ng/ml show higher G-grades than patients with a serum level of integrin avb6 < 4 ng/ml.

### Example 1: ELISA

Serum samples from healthy control patients (KO, n=10), patients with acute inflammatory bowel disease (IBD, B1, n=20), patients with fistulising Crohn's disease (B3; n=20) and colorectal carcinoma (CoCa, n=80) were analysed by ELISA. As shown in figure 1, the mean value of integrin αvβ6 in the serum of healthy control patients is almost zero, meaning it is not detectable in this population. A similar result can be observed for IBD patients featuring an inflammatory disease phenotype. In this group, the mean value of the analysed serum samples is also almost zero. In contrast, in patients with colorectal carcinoma, integrin αvβ6 can be detected in the serum, indicated by the clearly elevated mean value of integrin αvβ6 in this group.

### Example 2: Immunohistochemistry

Methods: Expression of ITGB6 and co-localization with matrix metalloproteinase 9 (MMP9) and fibronectin in liver metastasis (n=13) and primary tumors (n=10) of colorectal carcinoma (CRC) patients were examined by immunohistochemistry (IHC). Integrin alpha (v)beta6 (ITGB6) serum levels in well-characterized CRC patients (n=64) were assessed by ELISA. The correlation with clinical and pathologic characteristics was analyzed. mRNA levels were assessed by real-time RT-PCR in HT29-intestinal epithelial cells (IEC).

Results: By IHC, considerable ITGB6 staining was found along the invasion front of primary CRC tissue and the CRC metastasis in liver. IHC revealed co-expression of ITGB6 stained cells with MMP9, which is activated by ITGB6, as well as with fibronectin, the ligand of ITGB6, at the invasion front.

ITGB6 was detectable in the serum of CRC patients showing a clear cutoff value of 4 ng/ml ITGB6. Higher serum levels were associated with worse survival (2.51 ± 1.44 vs. 4.43 ± 3.71 years). All of the patients with levels higher than 4 ng/ml ITGB6 revealed T stage ≥ 2, G stage ≥ 2, indicative for infiltrative and de-differentiated tumors, as well as 100 % metastatic disease (M).

To assess how ITGB6 expression is regulated, HT29-IECs were treated with tumor necrosis factor (TNF, 48 h), a known mediator of EMT and/or epidermal growth factor (EGF; 24 h). TNF-treatment resulted in increased mRNA levels of ITGB6 (p<0.01), EGF receptor (EGFR; p<0.05), Ets-1 transcription factor, the suggested regulator of ITGB6 (p<0.01), as well as elevated vimentin (p<0.01), but decreased E-cadherin (p<0.001), indicating EMT. Stimulation with EGF resulted in decreased ITGB6 (p<0.05) and E-cadherin (p<0.05), but increased EGFR (p<0.05) and vimentin (p<0.001) mRNA levels. EGF treatment of TNF-pretreated cells impaired the TNF-induced rise in ITGB6 (p<0.01) and Ets-1 (p<0.05), but enhanced the EGFR mRNA level (p<0.001).

Conclusions: In summary, ITGB6 is strongly expressed at the invasion front of CRC primary tumours and metastases suggesting a crucial role for ITGB6 in CRC progression. Elevated ITGB6 serum levels correlate with poor survival and advanced TNM stage indicating advanced disease and demonstrate the utility of ITGB6 as a novel CRC serum tumour marker in clinical practice.

### Materials and Methods

### Serum samples

Serum samples were obtained from healthy control patients (KO, n=10), patients with active inflammatory IBD (B1, n=20), patients with fistulising Crohn's disease (B3; n=20) and colorectal carcinoma (CoCa, n=80) featuring any T, any N and any M status. Serum samples were collected from male and female patients. Written informed consent was obtained before specimen collection and studies were approved by the local ethics committee.

### Enzyme-linked immunosorbent assay (ELISA)

Serum samples were immediately frozen after sampling and stored at -80 C until further use. ELISA kit detecting human integrin αvβ6 (USCNK Life Sciences Inc., ELISA for Integrin β6, E92099Hu) was obtained from Chemie Brunschwig AG, Basel, Switzerland. Assays were performed according to the manufacturer's instructions using a sample volume of 50 µl. Absorbance at 450 nm was detected on a SpectraMax M2 Fluorescence Microplate reader using SoftMax Pro v5 Software (Molecular Devices, Sunnyvale, CA).

### Statistical analysis

Data are presented as means +/- S.E.M. for a series of n experiments.

Data analysis was performed using SPSS statistics software.

## Claims

1. A method for diagnosis of colorectal carcinoma in a patient, comprising detecting, in a sample obtained from said patient, an indicator protein selected from the group comprised of integrin subunit alpha(v), integrin subunit beta(6) and a heterodimer comprising the integrin subunits alpha(v) and beta(6).

2. The method according to claim 1, comprising determining an amount of said indicator protein.

3. A method according to any of the previous claims, comprising the steps of:
- contacting, in a first contacting step, said sample with a first ligand specifically reactive to said indicator protein;
- determining, in a quantification step, said amount of said indicator protein bound to said first ligand.

4. A method according to claim 2 or 3, further comprising comparing said amount to a standard.

5. A method according to claim 3 or 4, wherein said first ligand is attached to a surface.

6. A method according to any of claims 3 to 5, wherein said quantification step comprises the steps of contacting, in a second contacting step, said indicator protein with a second ligand specifically reactive to said indicator protein and determining an amount of said second ligand bound to said indicator protein.

7. The method according to claim 6, wherein said second ligand is attached or specifically attachable to an enzyme activity or a detectable label.

8. The method according to claim 7, wherein said detectable label is an optical label.

9. A method according to any of the previous claims wherein an amount of said indicator protein above 4 ng/ml is assigned to a high probability of said patient developing or having developed metastatic colorectal carcinoma, undifferentiated tumour or advanced colorectal carcinoma.

10. The use of a device comprising a surface, said surface comprising a ligand reactive to an indicator protein selected from the group comprised of integrin subunit alpha(v), integrin subunit beta(6) and a heterodimer comprising the integrin subunits alpha(v) and beta(6)attached to said surface, for the diagnosis of colorectal carcinoma.

11. The use of a kit of parts comprising
a. a device comprising a surface, said surface comprising a ligand reactive to an indicator protein selected from the group comprised of integrin subunit alpha(v), integrin subunit beta(6) and a heterodimer comprising integrin subunits alpha(v) and beta(6) attached to said surface, for the diagnosis of colorectal carcinoma, and
b. a second ligand reactive to said indicator protein in diagnosis of colorectal carcinoma.
